(19) Europäisches Patentamt
European Patent Office
Office européen des brevets

(11) Veröffentlichungsnummer: **0 192 932**
**B1**

(12) # EUROPÄISCHE PATENTSCHRIFT

(45) Veröffentlichungstag der Patentschrift: **12.07.89**

(51) Int. Cl.⁴: **C 08 B 37/08**, A 61 K 7/48

(21) Anmeldenummer: **86100300.2**

(22) Anmeldetag: **10.01.86**

(54) **Kosmetische Mittel auf der Basis von quaternären Chitosanderivaten, neue quaternäre hydroxyethyl-substituierte Chitosanderivate, sowie Verfahren zu ihrer Herstellung.**

(30) Priorität: **29.01.85 DE 3502833**

(43) Veröffentlichungstag der Anmeldung:
**03.09.86 Patentblatt 86/36**

(45) Bekanntmachung des Hinweises auf die Patenterteilung:
**12.07.89 Patentblatt 89/28**

(84) Benannte Vertragsstaaten:
**AT BE CH DE FR GB IT LI LU NL SE**

(56) Entgegenhaltungen:
**EP-A-0 115 574**

**CHEMICAL ABSTRACTS, Band 98, Nr. 22, Mai 1983, Nr. 181486e, Columbus, Ohio, US; & JP - A - 57 180 602 (NIPPON SODA CO., LTD.) 06-11-1982**

**Die Akte enthält technische Angaben, die nach dem Eingang der Anmeldung eingereicht wurden und die nicht in dieser Patentschrift enthalten sind.**

(73) Patentinhaber: **Wella Aktiengesellschaft, Berliner Allee 65, D-6100 Darmstadt (DE)**

(72) Erfinder: **Lang, Günther, Dr., Auf der Roten Erde 10 B, D-6107 Reinheim 5 (DE)**
Erfinder: **Wendel, Harald, Grabengasse 3, D-6105 Ober- Ramstadt (DE)**
Erfinder: **Konrad, Eugen, Mecklenburger Strasse 101, D-6100 Darmstadt (DE)**

**EP 0 192 932 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die Erfindung betrifft kosmetische Mittel zur Behandlung von Haaren oder der Haut mit einem Gehalt an neuen makromolekularen quaternären, vom Chitosan abgeleiteten Verbindungen in einer geeigneten Kosmetikgrundlage.

Die Erfindung betrifft weiterhin die neuen quaternären Chitosanderivate sowie Verfahren zu ihrer Herstellung.

Es ist bereits bekannt, in kosmetischen Mitteln, insbesondere für die Behandlung von Haaren, kationaktive Polymere, insbesondere Polymere, die quaternäre Ammoniumgruppen aufweisen, als Konditionierungsmittel einzusetzen. Auf Grund einer Wechselwirkung zwischen ihren Ammoniumgruppen und den anionischen Gruppen des Haares besitzen die kationaktiven Polymere eine große Affinität zur Keratinfaser.

Es wurde festgestellt, daß der Einsatz derartiger kationaktiver Polymere in solchen kosmetischen Mitteln zahlreiche Vorteile ergibt; die Entwirrung der Haare sowie deren Behandlung wird erleichtert, und weiterhin wird dem Haar Sprungkraft und Glanzwirkung verliehen. Durch die Affinität zum Keratin neigen diese Polymere jedoch bei wiederholter Anwendung zur Ansammlung auf den Haaren, so daß diese schwerer werden, was im Endeffekt unerwünscht ist.

Weiterhin ergeben sich bei synthetischen Polymeren Probleme wegen der physiologischen Wirkung von eventuell vorhandenen Monomerspuren, die nur schwer aus dem Polymer entfernbar sind.

Man hat bereits versucht, diese vorerwähnten Nachteile dadurch zu beheben, daß wasserlösliche Salze des Chitosans, eines durch Entacetylierung von Chitin herstellbaren Polyglucosamins, in derartigen kosmetischen Mitteln Anwendung finden. In diesem Zusammenhang wird Bezug genommen auf die eigene europäische Patentschrift EP-B-0 002 506 sowie die eigene deutsche Patentschrift DE-B-2 627 419.

In gleicher Weise wie bei der Mehrzahl der kationaktiven Polymere mit quaternären Gruppierungen ergibt Chitosan ebenfalls häufig den Nachteil, daß es mit den anionaktiven oberflächenaktiven Agentien, die üblicherweise in kosmetischen Mitteln zur Behandlung von Haaren, insbesondere in Shampoos, Anwendung finden, wenig verträglich ist. Es ist daher notwendig, das Chitosan in separaten Behandlungen, nämlich vor und/oder nach der Shampoonierung zur Einwirkung zu bringen.

Das Chitosan erweist sich weiterhin in neutralem und alkalischem Medium als praktisch unlöslich, so daß seine Anwendung beispielsweise in alkalischen Dauerwellmitteln oder Haarfärbemitteln nicht möglich ist.

Durch Einsatz von quaternären Chitosanderivaten gemäß unserer eigenen EP-A 0 115 574 anstelle von Chitosansalzen lassen sich die meisten der vorstehend erwähnten Nachteile vermeiden. Die aus der EP-A 0 115 574 bekannten Chitosanderivate neigen jedoch bei wiederholter Anwendung, wenn auch weniger stark als die üblicherweise in Haarbehandlungsmitteln verwendeten kationischen Polymere (beispielsweise kationische Cellulosederivate vom Polymer® JR-Typ) so doch merklich, zur Ansammlung auf den Haaren, wodurch diese belastet werden und rasch nachfetten. Hierdurch erhält das Haar einen unerwünschten schmierigen und fettigen Griff sowie ein ungepflegtes Aussehen. Weiterhin ist die Herstellung der dort beschriebenen quaternären Chitosanderivate sehr kostenintensiv, da das für die Herstellung dieser chitosanderivate erforderliche Glycidol (2,3-Epoxy-1-propanol) ein teurer, großtechnisch nicht hergestellter und zudem hydrolyseempfindlicher Rohstoff ist.

Aufgabe der Erfindung ist es deshalb, Mittel zur verfügung zu stellen, mit denen sich die vorstehend genannten Nachteile - insbesondere die starke Belastung der Haare bei wiederholter Anwendung - vermeiden lassen.

Bei der Fortführung der Untersuchungen mit Chitosan und den davon abgeleiteten Verbindungen wurde nunmehr gefunden, daß bestimmte quaternäre Chitosanderivate die vorstehend aufgeführten Nachteile nicht aufweisen und zudem wesentlich kostengünstiger als die aus der EP-A 0 115 574 bekannten Chitosanderivate erhältlich sind.

Die neuen Chitosanderivate besitzen eine ausgezeichnete haarkonditionierende Wirkung ohne das Haar zu belasten. Im Gegensatz zu synthetischen Polymeren mit endlichen Restmonomerengehalten sind die neuen quaternären Chitosanderivate physiologisch unbedenklich und biologisch abbaubar.

Weiterhin besitzen diese Chitosanderivate hervorragende Filmeigenschaften sowie eine gute Aniontensidverträglichkeit und sind sowohl in Wasser als auch in Alkoholen löslich.

Mit diesen quaternären Chitosanderivaten lassen sich somit kosmetische Mittel zur Behandlung von Haaren oder der Haut herstellen, die sich durch überraschend vorteilhafte Eigenschaften auszeichnen und die dadurch gekennzeichnet sind, daß sie in einer geeigneten Kosmetikgrundlage eine quaternäre makromolekulare, vom Chitosan abgeleitete polymere Verbindung der allgemeinen Formel I

$$HO[C_6H_{11-m}NO_4(R^1)_m(R^2)_n(R^3)_q]_pH, \qquad (I)$$

wobei

m jeden beliebigen Zahlenwert von 0 bis 0,5 bedeutet,
n jeden beliebigen Zahlenwert von 0,01 bis 6 bedeutet,
q ein beliebiger Zahlenwert von 0,005 bis 3,0 ist,
p eine ganze Zahl von 10 bis 50 000 bedeutet,
$R^1$ ein Acetyl-Rest ist,
$R^2$ einen zweiwertigen Rest

$-CH_2-CH_2-O-$

darstellt und

$R^3$ ein zweiwertiger Rest

$$\begin{array}{l} -CH_2-CH-O- \\ \quad\quad\;\; | \\ \quad\quad CH_2 \\ \quad\quad\;\; | \\ (R^4)_3N^+ \;\; X^- \end{array} \qquad \text{oder} \qquad \begin{array}{l} -CH-CH_2-O- \\ \;\;\; | \\ \; CH_2 \\ \;\;\; | \\ (R^4)_3N^+ \;\; X^- \end{array}$$

mit

$R^4$ = $C_1$- bis $C_4$-Alkyl und

X = Cl, Br, J oder $CH_3SO_4$ ist, enthalten.

Die erfindungsgemäßen, quaternären Chitosanderivate der Formel I enthaltenden Mittel eignen sich ganz allgemein zur Behandlung der Haut und/oder der Haare. Sie können beispielsweise vorliegen als Haar- und/oder Körperwaschmittel, Tönungsshampoos, Frisiercremes, Frisierlotionen, Fönlotionen, Mittel zur Festigung der Frisur, Waschlotionen, Haarkuren, Mittel gegen Kopfschuppen, Mittel zur permanenten Haarverformung, Mittel zur Färbung oder Entfärbung von Haaren, Mittel zum Auftragen vor oder nach der Haarfärbung und als kosmetische Mittel zur Pflege, zum Schutz oder zur Reinigung der Haut wie Gesichtswässer, Rasierwässer, Feuchthaltecremes, Coldcremes, Körperlotionen, Sonnenschutzmittel oder auch Make-up-Präparate wie Schminkcremes und Rouges.

Der Gehalt der erfindungsgemäßen kosmetischen Mittel an den neuen Chitosanderivaten der Formel I liegt zweckmäßig bei 0,05 bis 10 Gew.-%, vorzugsweise bei 0,05 bis 3,0 Gew.-%.

Die kosmetischen Mittel gemäß der vorliegenden Erfindung können zusätzlich zu dem neuen Chitosanderivat der Formel I zur Herstellung einer Kosmetikgrundlage alle diejenigen Bestandteile enthalten, die in Haar- und Hautbehandlungsmitteln üblicherweise eingesetzt werden, insbesondere anionische, kationische, amphotere, zwitterionische oder nichtionische oberflächenaktive Tenside, Schaumsynergisten, Stabilisatoren, Sequestriermittel, Pigmente, Verdicker, Emulgatoren, Pufferstoffe, Konservierungsmittel, Farbstoffe, Parfümöle, bekannte kosmetische Polymere wie anionische, nichtionische, kationische oder amphotere Polymere, Naturstoffe, kosmetische Öle, Fettalkohole, Wachse, Schaumstabilisatoren, Wirkstoffe gegen Kopfschuppen, Reduktionsmittel und Treibgase.

Die erfindungsgemäßen kosmetischen Mittel weisen in bevorzugter Weise einen pH-Wert von 2 bis 11 auf und können in Form wäßriger, alkoholischer oder wäßrig-alkoholischer Zubereitungen, z. B. mit einem Alkohol mit 1 bis 4 Kohlenstoffatomen, als Lösungen, als Cremes, als Gele, als Dispersionen oder als Emulsionen vorliegen. Ebenfalls ist es möglich, diese Mittel in Form der alkoholischen oder wäßrig-alkoholischen Lösung mit Hilfe eines Zerstäubers beziehungsweise anderer geeigneter Sprühvorrichtungen oder im Gemisch mit üblichen unter Druck verflüssigten Treibgasen als Aerosolhaarspray aus einem Druckbehälter zu versprühen.

Wenn es sich bei den erfindungsgemäßen kosmetischen Mitteln um Mittel zur Festigung der Frisur, wie flüssige Haarfestiger oder Haarsprays, handelt, dann liegen sie üblicherweise als wäßrig-alkoholische Lösungen vor, die durch einen Gehalt an quaternären Chitosanderivaten der vorstehend genannten Formel I gekennzeichnet sind. Hierbei können die quaternären Chitosanderivate selbst als filmbildendes beziehungsweise festigendes Harz eingesetzt werden. Es können jedoch auch zusätzlich andere filmbildende natürliche oder synthetische Polymere in dem erfindungsgemäßen Haarfestigungsmittel enthalten sein. Als natürliche Polymere kommen beispielsweise Schellack, Alginate, Gelatine, Pektine und Cellulosederivate in Betracht. Von den synthetischen Polymeren finden z. B. Polyvinylpyrrolidon, Polyvinylacetat, Polyacrylverbindungen, wie Acrylsäure- oder Methacrylsäurepolymerisate, basische Polymerisate von Estern aus Acrylsäure oder Methacrylsäure mit Aminoalkoholen beziehungsweise die Salze oder Quaternisierungsprodukte dieser basischen Polymerisate, Polyacrylnitril sowie Co- oder Terpolymerisate aus derartigen Verbindungen, beispielsweise Polyvinylpyrrolidon-Vinylacetat, Verwendung. Die Mittel weisen dann insbesondere einen pH-Wert zwischen 6 und 8 auf. Solche Mittel zur Festigung der Frisur enthalten üblicherweise filmbildende Polymere in einer Gesamtmenge von etwa 0,05 bis 3,0 Gew.-%. Enthalten die Mittel neben den quaternären Chitosanderivaten der Formel I noch andere filmbildende Polymere, so reduziert sich der Gehalt an quaternären Chitosanderivaten entsprechend.

Als Alkohole kommen insbesondere die für kosmetische Zwecke üblicherweise verwendeten niederen Alkohole wie Ethanol und Isopropanol in Betracht.

Die erfindungsgemäßen Mittel zur Festigung der Frisur können weiterhin die üblichen Zusätze wie beispielsweise Parfümöl, Bakterizide oder Fungizide, kämmbarkeitsverbessernde Substanzen und so weiter enthalten.

Die erfindungsgemäßen Mittel zur Festigung der Frisur können gegebenenfalls durch einen Gehalt an kosmetischen Farbstoffen das Haar gleichzeitig färben oder tönen. Derartige Präparate sind unter anderem als Farbfestiger oder Tönungsfestiger im Handel bekannt. Sie enthalten zusätzlich übliche für Haarfestiger bekannte Farbstoffe wie beispielsweise aromatische Nitrofarbstoffe (zum Beispiel 1,4-Diamino-2-nitrobenzol),

Azofarbstoffe (zum Beispiel C.I. Acid Brown 4), Anthrachinonfarbstoffe (zum Beispiel C.I. Disperse Violet 4), und Triphenylmethanfarbstoffe (zum Beispiel C.I. Basic Violet 1), wobei die Farbstoffe dieser Klassen je nach der Art ihrer Substituenten sauren, nichtionogenen oder basischen Charakter haben können. Ihre Gesamtkonzentration in diesen Präparaten beträgt üblicherweise etwa 0,01 bis 2,0 Gew.-%.

Die erfindungsgemäßen Mittel zur Festigung der Frisur weisen bei gleich guter Festigung des Haares gegenüber üblichen Mitteln eine verbesserte Substantivität zum Haar, eine besonders gute Kämmbarkeit und einen guten Griff des Haares im nassen Zustand sowie einen besonders angenehmen Griff des Haares im getrockneten Zustand auf.

Wenn die erfindungsgemäßen Mittel Haarwaschmittel darstellen, liegen sie in Form wäßriger Lösungen oder Emulsionen vor und enthalten neben dem neuen Chitosanderivat zumindest ein anionisches, kationisches, nichtionisches oder amphoteres Tensid.

In diesem Haarwaschmittel liegt die Konzentration des Tensides im allgemeinen zwischen 3 und 50 Gew.-% und vorzugsweise zwischen 3 und 25 Gew.-%, bezogen auf das Gesamtgewicht des Mittels, wobei der pH-Wert im allgemeinen zwischen 3 und 9 und vorzugsweise zwischen 6 und 7 liegt.

Die erfindungsgemäßen Mittel, die in Form von Haarwaschmitteln vorliegen, enthalten im allgemeinen verschiedene Zusatzstoffe, insbesondere Parfüms, Konservierungsstoffe, Verdicker, Schaumstabilisatoren, Puffersubstanzen, kosmetische Harze, Pigmente und Farbstoffe.

Unter den Schaumstabilisatoren können die Fettamide und insbesondere die Mono- oder Diethanolamide von Copra-Fettsäuren, Lauryl- oder Ölsäuremono- oder -diethanolamid, die zweckmäßigerweise in Mengen von 1 bis 10 und vorzugsweise 1 bis 3 Gew.-%, bezogen auf das Gesamtgewicht des Mittels Verwendung finden, angeführt werden.

Unter den Verdickern können insbesondere die Acrylpolymere und die Cellulosederivate wie Carboxymethylcellulose, Hydroxypropylmethylcellulose, Hydroxyethylcellulose angeführt werden. Die Verdicker liegen im allgemeinen in einem Anteil von 0,1 bis 5 Gew.-% vor.

Unter den Tensiden oder oberflächenaktiven Agentien, die in Kombination mit den neuen quaternären Chitosanderivaten verwendet werden, können beispielsweise die folgenden genannt werden:

a) die anionischen oberflächenaktiven Agentien, wie beispielsweise die Alkali- oder Erdalkali-, Ammonium- oder Alkanolaminsalze von Alkansulfonaten, Alkylsulfaten und Alkylethersulfaten, die $C_{12}$-$C_{18}$-Alkyl- und insbesondere $C_{12}$-$C_{14}$-Alkyl-Sulfatnatriumsalze oder Triethanolaminsalze, die Natrium- oder Triethanolaminsalze von Lauryl- oder Tetradecylethersulfaten, das Dinatriumsalz des Sulfosuccinhalbesters von Alkanolamiden, die Seifen und die Polyethercarbonsäuren;

b) die nichtionischen oberflächenaktiven Agentien, wie beispielsweise oxethylierte Fettalkohole mit 12 bis 18 Kohlenstoffatomen, zum Beispiel mit bis zu 40 Mol Ethylenoxid pro Mol Fettalkohol oxethylierter Laurin-, Tetradecyl-, Cetyl-, Olein-, Palmitin- und Stearinalkohol, allein oder im Gemisch, die Fettalkohole von oxethyliertem Lanolin oder oxethyliertes Lanolin; Polyglycerylether von gesättigten oder ungesättigten Fettalkoholen und Alkylphenolen mit 8 bis 30 Kohlenstoffatomen im Alkylrest und 1 bis 10 Glyceryleinheiten im Molekül sowie Fettsäurealkanolamide;

c) die kationischen oberflächenaktiven Agentien, wie beispielsweise das Dilauryldimethylammoniumchlorid, die Chloride oder Bromide von Alkyldimethylbenzylammonium, die Alkyltrimethylammoniumsalze, beispielsweise Cetyltrimethylammoniumchlorid oder -bromid, Tetradecyltrimethylammoniumchlorid oder -bromid, die Alkyldimethylhydroxyethylammoniumchloride oder -bromide, die Dialkyldimethylammoniumchloride oder -bromide, Alkylpyridiniumsalze, beispielsweise Cetylpyridinumchlorid, die Alkylamidethyltrimethylammoniumethersulfate, Imidazolinderivate, Verbindungen mit kationischem Charakter wie Aminoxide, beispielsweise Alkyldimethylaminoxide oder Alkylaminoethyldimethylaminoxide,

d) die amphoteren oder zwitterionischen oberflächenaktiven Agentien wie beispielsweise die Carboxylderivate von Imidazol, die N-Alkylbetaine, die N-Alkylsulfobetaine, die N-Alkylaminobetaine, die N-Alkylaminopropionate, die Alkyldimethylammoniumacetate beziehungsweise die $C_{12}$-$C_{18}$-Alkyldimethylcarboxymethylammoniumsalze.

Die erfindungsgemäßen kosmetischen Mittel können auch Cremes oder Lotionen zur Verwendung als Haarkur oder Hautpflegemittel darstellen. Sie liegen dann meist in Form von Öl-in-Wasser- oder Wasser-in-Öl-Emulsionen oder Suspensionen vor und enthalten zusätzlich zu den neuen Chitosanderivaten der Formel I kationische, nichtionogene, amphotere oder anionische Emulgatoren sowie als Bestandteil der Ölphase zum Beispiel Fettalkohole, Fettsäureester- oder amide, weiterhin Parfümöle, Vaseline, Wollfettalkohol oder feste oder flüssige Paraffine.

Wenn die erfindungsgemäßen Mittel Haartönungs- oder Haarfärbemittel darstellen, so liegen sie ebenfalls bevorzugt in Form von Cremes oder Lotionen vor und enthalten zusätzlich übliche Haarfarbstoffe aus der Gruppe der aromatischen Nitrofarbstoffe, Azofarbstoffe, Anthrachinonfarbstoffe, Triphenylmethanfarbstoffe oder auch Oxidationsfarbstoffe, beispielsweise aus der Gruppe der aromatischen Diamine beziehungsweise Aminophenole. Weiterhin können diese Mittel gegebenenfalls Alkalisierungsmittel, Antioxidantien sowie weitere für solche Mittel übliche kosmetische Zusätze und Hilfsstoffe enthalten.

Die erfindungsgemäßen Mittel können auch Dauerverformungsmittel oder Fixiermittel für Haare darstellen.

Sie enthalten dann zusätzlich zu den genannten Chitosanderivaten der Formel I Reduktionsmittel, wie zum Beispiel Thioglykolsäure und Ammoniumsulfit beziehungsweise Oxidationsmittel, wie zum Beispiel Wasserstoffperoxid oder Natriumbromat sowie gegebenenfalls Alkalisierungsagentien beziehungsweise Peroxidstabilisatoren, zum Beispiel Phosphorsäure, und andere kosmetische Hilfsstoffe und Zusatzstoffe wie beispielsweise Parfümöle, Riechstoffe, Pflegestoffe und Farbstoffe.

Wie bereits erwähnt wurde, können die erfindungsgemäßen kosmetischen Mittel auch zur Behandlung der Haut verwendet werden.

In der Tat erleichtern diese kosmetischen Mittel die Befeuchtung der Haut und verhindern das Austrocknen. Diese Mittel verleihen der Haut weiterhin eine hervorragende Weichheit im Griff.

Die erfindungsgemäßen kosmetischen Mittel liegen hierzu vorzugsweise in Form von Cremes, Gelen, Emulsionen oder wäßrigen, alkoholischen oder wäßrig-alkoholischen Lösungen vor, die das Chitosanderivat der Formel I in einer Konzentration von 0,1 bis 10 Gew.-% und vorzugsweise von 0,2 bis 6 Gew.-% enthalten.

Die im allgemeinen in diesen Kosmetikzubereitungen enthaltenen Hilfsstoffe sind beispielsweise Duftstoffe, Farbstoffe, Konservierungsmittel, Verdickungsmittel, Sequestriermittel, Emulgiermittel, Sonnenschutzfilter etc.

Diese Zubereitungen für die Hautpflege liegen insbesondere in Form von Cremes oder Lotionen zur Pflege der Hände oder des Gesichts oder in Form von Sonnenschutzcremes, gefärbten Cremes, Abschminkmilchprodukten, Schaumbad- und Duschbad-Präparaten oder auch in Form von Deodorierzubereitungen vor.

Diese Zubereitungen werden unter Anwendung klassischer Verfahrensweisen hergestellt.

Beispielsweise kann man zur Bildung einer Creme eine wäßrige Phase, die das erfindungsgemäße Chitosanderivat und gegebenenfalls andere Bestandteile oder Hilfsstoffe gelöst enthält, und eine ölige Phase emulgieren.

Für die ölige Phase kann man verschiedenartige Verbindungen verwenden, beispielsweise Paraffinöl, Vaselinöl, Süßmandelöl, Avocadoöl, Olivenöl, Fettsäureester, wie Glycerylmonostearat, Ethylpalmitat oder Isopropylpalmitat oder Alkylmyristate, wie Propylmyristat, Butylmyristat oder Cetylmyristat. Man kann sie auch mit Fettsäurealkoholen, wie Cetylalkohol oder Wachsen, beispielsweise Bienenwachs, versetzen.

Die Chitosanderivate der Formel I können in den Kosmetikzubereitungen für die Hautpflege entweder als Hilfsstoff oder als Hauptwirkstoff enthalten sein.

Die in den erfindungsgemäßen kosmetischen Mitteln enthaltenen neuen Chitosan-Derivate leiten sich von Chitosan ab, einem Material, das durch Entacetylierung von Chitin, einem natürlich vorkommenden Acetylglucosamin, erhalten wird.

Das Chitosan ist im neutralen und alkalischen Medium unlöslich, es bildet jedoch auf Grund seiner chemischen Natur im sauren Medium mit organischen und anorganischen Säuren Salze, die beispielsweise in der Papier- und Textilindustrie als Additive Verwendung finden sowie weiterhin als Koagulanzien für Suspensionen, als Chelatbildner für Schwermetallionen sowie in der Medizin und in der Kosmetik benutzt werden (siehe in diesem Zusammenhang die Veröffentlichung von Muzarelli: "Chitin", Pergamon Press, 1977).

Es sind auch bereits einige wasserlösliche Chitosanderivate bekannt, beispielsweise Carboxymethylchitosan, Sulfoethylchitosan (siehe Nud'ga, Plisko und Danilov, Zhur. Prikl.Khim. 47, (1974) 872-875). Diese wasserlöslichen Chitosanderivate sind jedoch in ihrem ionischen Charakter verändert oder aber physiologisch bedenklich (Epichlorhydrinchitosan, Veröffentlichung von Noguchi, Arato und Komai, Kogyo Kagaku Zasshi 72, (1969) 796-799 und japanische Patentanmeldung Nr. 46-39 322, H. Haga).

Diese vorerwähnten polymeren Verbindungen erfordern außerdem für ihre technische Herstellung relativ aufwendige Verfahren.

Weiterhin berichtet unsere eigene EP-A 0 115 574 von wasserlöslichen N-substituierten quaternären Chitosanderivaten, die durch Umsetzung von Chitosan mit Glycidyltrialkylammoniumhalogeniden und Glycidol (2,3-Epoxy-1-propanol) in Gegenwart eines geeigneten Lösungsmittels erhalten werden. Die rasche Hydrolyse des Glycidols in Gegenwart von Wasser, sein hoher Preis und die Tatsache, daß Glycidol nicht großtechnisch hergestellt wird und somit nicht in beliebiger Menge verfügbar ist, verteuern jedoch das Verfahren zur Herstellung dieser Chitosanderivate.

Es wurde nunmehr gefunden, daß sich durch Umsetzung von Chitosan mit einem Glycidyltrialkylammoniumhalogenid (Oxyranmethanammonium-N,N,N-trialkylhalogenid) der Formel

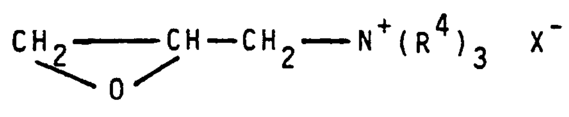

($R^4$ = $C_1$- bis $C_4$-Alkyl; X = Cl, Br, J oder $CH_3SO_4$)

sowie zusätzlich mit Ethylenoxid in einfacher Weise quaternäre Chitosanderivate mit hoher Substantivität, unter anderem zu Haarkeratin, herstellen lassen.

Die neuen quaternären makromolekularen, vom Chitosan abgeleiteten polymeren Verbindungen sind gekennzeichnet durch die allgemeine Formel I

$$HO[C_6H_{11-m}NO_4(R^1)_m(R^2)_n(R^3)_q]_pH, \qquad (I)$$

wobei

m jeden beliebigen Zahlenwert von 0 bis 0,5 bedeutet,
n jeden beliebigen Zahlenwert von 0,01 bis 6 bedeutet,
q ein beliebiger Zahlenwert von 0,005 bis 3,0 ist,
p eine ganze Zahl von 10 bis 50 000 bedeutet,
$R^1$ ein Acetyl-Rest ist,
$R^2$ einen zweiwertigen Rest

$-CH_2-CH_2-O-$

darstellt und
$R^3$ ein zweiwertiger Rest

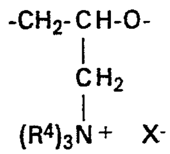

oder

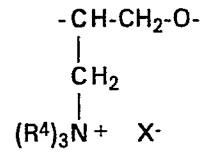

mit
$R^4 = C_1$- bis $C_4$-Alkyl und
$X = Cl, Br, J$ oder $CH_3SO_4$ ist,
wobei der Ausdruck in eckigen Klammern sich wiederholende substituierte Glucosamin-Monomereinheiten darstellen soll.

Die neuen, quaternären Stickstoff enthaltenden Chitosanderivate werden erfindungsgemäß hergestellt, indem man ein Chitosan, bestehend aus zu 50 - 100 % entacetyliertem Chitin, in Gegenwart eines Lösungsmittels mit einem Glycidyltrialkylammoniumhalogenid und Ethylenoxid in geeignetem Verhältnis zur Umsetzung bringt. Bei der Umsetzung kann auch auf ein Lösungsmittel verzichtet werden, wobei dann ein Überschuß an Ethylenoxid als Lösungsmittel dient.

Geeignete Glycidyltrialkylammoniumhalogenide sind zum Beispiel Glycidyltrimethylammoniumchlorid und Glycidyltriethylammoniumchlorid. Die den quaternären Stickstoff enthaltenden Epoxide können aber auch in situ durch Umsetzung der entsprechenden Chlorverbindung mit basischen Katalysatoren (beispielsweise Umsetzung von 1-Chlor-3-trimethylammonium-propanol-2-chlorid mit Natronlauge) vor oder während der Umsetzung mit dem Chitosan erzeugt werden.

Die Umsetzung des Chitosans mit Ethylenoxid und dem quaternären Epoxyd kann in einfacher Weise durch gleichzeitige Umsetzung mit beiden Komponenten erfolgen, es ist jedoch auch möglich, die Umsetzung in zwei Stufen durchzuführen. So kann zunächst das Chitosan mit dem Ethylenoxid und das erhaltene Produkt mit dem quaternären Epoxid oder das Chitosan zunächst mit dem quaternären Epoxid und sodann mit Ethylenoxid umgesetzt werden.

Hierzu wird zweckmäßig das Chitosan in feingepulverter Form eingesetzt. Die Umsetzung selbst kann bei Temperaturen zwischen 10 und 100°C, vorzugsweise zwischen 50 und 80°C, erfolgen, und diese Temperatur wird zweckmäßig unter Rühren 2 bis 100 h beibehalten. Die Reaktion kann mit oder ohne saure oder basische Katalysatoren in Gegenwart von Lösungsmitteln oder, bei Verwendung eines Überschusses an Ethylenoxid auch ohne ein besonderes Lösungsmittel erfolgen.

Vorzugsweise wird die Reaktion jedoch in Gegenwart von Wasser durchgeführt. Es hat sich dabei als vorteilhaft erwiesen, ohne zusätzliche Katalysatoren zu arbeiten, wobei das Verhältnis von Chitosan zu Wasser zwischen 1 : 0,05 und 1 : 100 und das Verhältnis von Chitosan zur Gesamtmenge aus dem Ethylenoxid und dem Glycidyltrialkylammoniumhalogenid (bezogen auf die Mole von substituierbaren Aminogruppen am Chitosan) zwischen 1 : 0,5 und 1 : 30, vorzugsweise zwischen 1 : 1 und 1 : 10, liegt. Das Verhältnis zwischen dem Ethylenoxid und dem Glycidyltrialkylammoniumhalogenid soll bei der Umsetzung, je nach dem gewünschtem Substitutionsgrad, zweckmäßigerweise zwischen 1 : 100 und 100 : 1, vorzugsweise zwischen 1 : 10 und 10 : 1, liegen.

Obwohl die Durchführung der Reaktion in Gegenwart von Wasser bevorzugt ist, kann sie auch unter Verwendung anderer Lösungsmittel, in denen mindestens eines der Reaktionsprodukte löslich ist, durchgeführt werden. Beispiele für solche Lösungsmittel sind Alkohole wie Ethanol, Methanol, Glykol und Glycerin sowie Ketone, beispielsweise Methylethylketon und Aceton.

Gemäß einer anderen Ausführungsform des erfindungsgemäßen Verfahrens zur Herstellung der Chitosanderivate der Formel I werden bei der Umsetzung zusätzlich organische oder anorganische Säuren oder Basen als Katalysatoren zugesetzt.

Für einen Einsatz als Katalysatoren geeignete Säuren sind zum Beispiel Salzsäure, Milchsäure und Ameisensäure. Beispiele für geeignete Basen sind Trialkylamine wie beispielsweise Trimethylamin, Triethylamin oder Trialkylolamine sowie Alkalihydroxide und Erdalkalihydroxide. Es ist grundsätzlich auch möglich, von wasserlöslichen Salzen des Chitosans, beispielsweise Chitosanlaktat, Chitosanacetat, Chitosanhydrochlorid, auszugehen. Hierbei kann jedoch als Nebenprodukt eine größere Menge der Glycerinester der verwendeten Säure entstehen, so daß die Reinigung der entstandenen Reaktionsprodukte erschwert wird.

Die Aufarbeitung der Reaktionsgemische kann beispielsweise in der Weise erfolgen, daß man das Lösungsmittel und gegebenenfalls überschüssiges Ethylenoxid im Vakuum aus der Reaktionsmischung abdestilliert.

Bei der Herstellung von wasserlöslichen quaternären Chitosanderivaten kann das Reaktionsprodukt in bevorzugter Weise in einem Überschuß an Wasser gelöst und durch Filtration oder Zentrifugieren vom nichtlöslichen Reaktionsrückstand abgetrennt werden. Als weitere Schritte zur Reinigung der Reaktionsprodukte können die wäßrigen Lösungen dialysiert werden und/oder gegebenenfalls nach Einengen der wäßrigen Lösungen durch Ausfällen in Aceton, Alkoholen oder anderen organischen Lösungsmitteln isoliert werden.

Gemäß einer besonders vorteilhaften Ausführungsform des erfindungsgemäßen Herstellungsverfahrens wird als Ausgangsmaterial durch Umfällen und Tiefgefrieren strukturell modifiziertes Chitosan eingesetzt. Mit einem solchen Ausgangsmaterial verläuft die Reaktion in besonders vorteilhafter Weise und in besonders guter Ausbeute.

Anhand der nachfolgenden Beispiele wird das erfindungsgemäße Verfahren zur Herstellung der neuen quaternären Chitosanderivate näher erläutert.

**Herstellungsbeispiele**

**Umsetzungen von Chitosan mit Glycidytrimethylammoniumchlorid und Ethylenoxid**

**Beispiel 1**

25 g (0,155 mol) Chitosan mit einer Grenzviskositätszahl von ($\eta$ = 1600 ml/g) und 76 % freies Amin, wurden mit der äquimolaren Menge Salzsäure in 5 l Wasser gelöst und anschließend durch Einstellen eines pH-Wertes von 9,5 mit Natronlauge ausgefällt.

Das ausgefällte und abgesaugte wasserhaltige Chitosan wurde im Autoklav (2 l Rauminhalt) mit 28 ml Ethylenoxid (0,564 mol) und 58,3 g einer 55 %-igen wäßrigen Lösung von Glycidyltrimethylammoniumchlorid 6 h lang bei 80°C gerührt.

Dabei wurde ein Druckanstieg auf 3,5 bar registriert. Das klar wasserlösliche Reaktionsprodukt, welches honigartige Konsistenz hatte, wurde durch Eintropfen in 20 l Aceton unter starkem Rühren ausgefällt, abgesaugt und anschließend bei 50°C im Vakuum getrocknet.

Es wurden 31,9 g quaternäres Chitosanderivat erhalten.

Kenndaten des quaternären Chitosanderivates:

| | |
|---|---|
| Grenzviskositätszahl: | 610 ml/g |
| Titrierbarer Stickstoff: | 3,08 mmol/g |
| Chloridbestimmung: | 2,01 mmol/g |
| Kolloid-Titration: | 2,49 mmol/g |

Substitutionsgrad daraus berechnet: q = 0,42; n = 0,87

Zur Berechnung des Substitutionsgrades wird zunächst die Molmenge an titrierbarem Stickstoff $N_t$ mmol/g durch nichtwäßrige Titration mit Perchlorsäure bestimmt. Das mittlere Molekulargewicht einer substituierten Chitosaneinheit berechnet sich daraus wie folgt:

$$\bar{M} = [g/mol] = \frac{1000}{N_t[\frac{mmol}{g}]}$$

Weiterhin gilt die Gleichung

$$\bar{M} = 161 + (q \cdot 152) + (n \cdot 44) + (m \cdot 42)$$

| | |
|---|---|
| - Molekulargewicht einer Chitosaneinheit = | 161 [g/mol] |
| - Molekulargewicht einer quaternären Gruppe $R^3$ aus Formel I ($R^4 = CH_3$; X = Cl) = | 152 [g/mol] |
| - Molekulargewicht einer Ethylenoxidgruppe ($R^2$ aus Formel I) = | 44 [g/mol] |
| - Molekulargewicht einer $CO\text{-}CH_2$-Gruppe = | 42 [g/mol] |

q = Substitutionsgrad, kationische Gruppen $R^3$
n = Substitutionsgrad, Ethylenoxid Gruppen $R^2$
z = Substitutionsgrad des verwendeten Chitosans mit Acetyl-Gruppen (bei 60 - 96 % freien Aminogruppen beträgt der Substitutionsgrad 0,04 - 0,4)

Zur Berechnung des Substitutionsgrades mit den kationischen Gruppen $R^3$ wird die Kolloid-Titration der kationischen Gruppen mit Polyvinylsulfat-Kaliumsalz herangezogen.

Es gilt: $q = \frac{[\text{Kolloid}] \cdot \bar{M}}{1000}$

Daraus läßt sich der Substitutionsgrad mit Ethylgruppen wie folgt berechnen:

$$n = \frac{\bar{M} - 161 - 152 \cdot q - 42 \cdot m}{44}$$

**Beispiel 2**

25 g Chitosan (0,155 mol) wurden wie in Beispiel 1 durch Umfällen zur Reaktion vorbereitet.

Danach wurde das Chitosan bei 80° C 6 Stunden lang unter Rühren im Autoklaven mit 30,2 ml Ethylenoxid und 47,1 g einer 55-%-igen wäßrigen Lösung von Glycidyltrimethylammoniumchlorid umgesetzt.

Das nicht vollkommen wasserlösliche Reaktionsprodukt wurde auf etwa 5 l mit Wasser verdünnt und über Plattenfilter klar filtriert. Danach wurde die Lösung im Rotationsverdampfer wieder auf etwa 2 l aufkonzentriert. Durch Eintropfen in 10 l Aceton wurde das Reaktionsprodukt ausgefällt, abgesaugt und bei 50° C im Vakuum getrocknet.

Es wurden 28,0 g eines quaternären Chitosanderivates erhalten.

Kenndaten des quaternären Chitosanderivates:

| | |
|---|---|
| Grenzviskositätszahl: | 513 ml/g |
| Titrierbarer Stickstoff: | 2,69 mmol/g |
| Chloridbestimmung: | 1,15 mmol/g |
| Kolloid-Titration: | 1,28 mmol/g |

Substitutionsgrad daraus berechnet: q = 0,48; n = 3,1

**Beispiel 3**

100 g Chitosan (0,62 mol) mit einer Grenzviskositätszahl von ($\eta$ = 140 ml/g) und einem freien Amin-Gehalt von 86 % wurden in 5 l Wasser dispergiert und mit der äquivalenten Menge Salzsäure gelöst. Anschließend wurde durch Einstellen eines pH-Wertes von 9,5 mit Natronlauge das Chitosan wieder ausgefällt.

Das umgefällte, wasserhaltige Chitosan wurde anschließend im Autoklaven 6 Stunden lang bei 80° C mit 112 ml Ethylenoxid und 233,2 g einer 55-%-igen wäßrigen Lösung von Glycidyltrimethylammoniumchlorid umgesetzt.

Das klar wasserlösliche Produkt wurde durch Eintropfen in 20 l Aceton unter starkem Rühren ausgefällt, abgesaugt und anschließend bei 50° C im Vakuum getrocknet.

Es wurden 126 g eines quaternären Chitosanderivates erhalten.

Kenndaten des Chitosanderivates

| | |
|---|---|
| Grenzviskositätszahl: | 46 ml/g |
| Titrierbarer Stickstoff: | 3,24 mmol/g |
| Chloridbestimmung: | 1,88 mmol/g |
| Kolloid-Titration: | 1,56 mmol/g |

Substitutionsgrad daraus berechnet: q = 0,48; n = 1,56

**Beispiele für kosmetische Mittel**

**Beispiel 4**

**Haarfestiger**

| | |
|---|---|
| 0,6 g | quaternäres Chitosanderivat nach Beispiel 3 ($\eta$ = 46 ml/g, Substitutionsgrad: kationische Gruppen 0,48; Hydroxyethyl-Gruppen 1,56) |
| 73,8 g | Wasser |
| 25,0 g | Isopropanol |
| 0,4 g | 10-%-ige Ameisensäure |
| 0,2 g | Parfümöl |
| 100,0 g | |

20 ml dieser Lösung wurden auf gewaschene, handtuchtrockene Haare verteilt, das Haar in üblicher Weise zur Frisur eingelegt und getrocknet. Bei guter Festigungswirkung zeigte das Haar, im Vergleich zu einem Haarfestiger auf der Basis von Chitosan/Ameisensäure, einen angenehmeren und weicheren Griff.

**Beispiel 5**

**Tönungsfestiger**

| | |
|---|---|
| 1,00 g | quaternäres Chitosanderivat nach Beispiel 2 (η = 513 ml/g, Substitutionsgrad: kationische Gruppen 0,48; Hydroxyethyl-Gruppen 3,1) |
| 1,00 g | Milchsäure |
| 0,10 g | Cetyltrimethylammoniumchlorid, 50-%-ige wäßrige Lösung |
| 0,05 g | Acid Brown 4 (C.I. 41 805) |
| 97,85 g | Wasser |
| 100,00 g | |

20 ml dieser Lösung wurden auf dem gewaschenen, handtuchtrockenen Haar verteilt und das Haar in üblicher Weise eingelegt und getrocknet. Das Haar zeigte anschließend eine leichte Rot-Braunfärbung.

**Beispiel 6**

**Tönungsfestiger**

| | |
|---|---|
| 0,60 g | quaternäres Chitosanderivat nach Beispiel 2 (η = 513 ml/g, Substitutionsgrad: kationische Gruppen 0,48; Hydroxyethyl-Gruppen 3,1) |
| 0,15 g | 1,4-Di(β -hydroxyethylamino)-2-nitro-5-chlorbenzol. |
| 25,00 g | Ethanol |
| 74,25 g | Wasser |
| 100,00 g | |

20 ml dieser Lösung wurden auf die gewaschenen, handtuchtrockenen Haare gegeben, sodann das Haar eingelegt und getrocknet. Die Haare waren rot-violett gefärbt und gefestigt.

**Beispiel 7**

**Anionisches Haarwaschmittel**

| | |
|---|---|
| 1,00 g | quaternäres Chitosanderivat nach Beispiel 1 (η = 610 ml/g), Substitutionsgrad: kationische Gruppen 0,42; Hydroxyethyl-Gruppen 0,87) |
| 40,00 g | Laurylalkoholdiglykolethersulfat-Natriumsalz, 28-%-ige wäßrige Lösung |
| 4,00 g | Natriumchlorid |
| 0,05 g | Farbstoff |
| 54,85 g | Wasser |
| 0,10 g | Formaldehyd, 25-%-ige wäßrige Lösung |
| 100,00 g | |

Es wurde ein klares Shampoo erhalten. Das damit gewaschene Haar war hinsichtlich Griff, Glanz und Kämmbarkeit ausgezeichnet konditioniert. Infolge der Verträglichkeit des quaternären Chitosanderivates mit Alkylethersulfat kann das vorstehend aufgeführte Shampoo erstellt werden, mit dem eine gleichzeitige Reinigung und Pflege des Haares möglich ist.

**Beispiel 8**

**Amphoteres, tönendes Haarwaschmittel**

| | |
|---|---|
| 2,00 g | quaternäres Chitosanderivat nach Beispiel 3 (η = 46 ml/g, Substitutionsgrad: kationische Gruppen 0,48; Hydroxyethyl-Gruppen 1,56) |
| 40,00 g | Dimethyl-carboxymethylen-propylenamido-stearatbetain, 35-%-ige wäßrige Lösung |
| 5,06 g | Ameisensäure, 10-%-ig |
| 3,50 g | Kokosfettsäurediethanolamid |
| 1,00 g | Pikraminsäure (C.I. 76 540), 1-%-ige wäßrige Lösung) |
| 48,44 g | Wasser, vollentsalzt |
| 100,00 g | |

Das Haar wurde mit etwa 20 g des obigen Mittels einshampooniert. Nach einer Einwirkungszeit von 5 bis 10 Minuten spülte man mit Wasser aus. Das Haar war gelborange getönt und ausgezeichnet konditioniert, besonders hinsichtlich Griff und Kämmbarkeit.

**Beispiel 9**

**Haarkurmittel, kationisch**

| | |
|---|---|
| 0,30 g | quaternäres Chitosanderivat nach Beispiel 3 (η = 46 ml/g, Substitutionsgrad: kationische Gruppen 0,48; Hydroxyethyl-Gruppen 1,56) |
| 4,00 g | Cetylstearylalkohol |
| 1,48 g | Milchsäure, 10-%-ig |
| 2,50 g | Kokos(pentaethoxy)methylammoniumchlorid |
| 1,00 g | Sorbitanmonopalmitat mit 20 Mol Ethylenoxid |
| 90,72 g | Wasser, vollentsalzt |
| 100,00 g | |

**Beispiel 10**

**Haarkurmittel, gelförmig**

| | |
|---|---|
| 2,10 g | quaternäres Chitosanderivat nach Beispiel 1 (η = 610 ml/g, Substitutionsgrad: kationische Gruppen 0,42; Hydroxyethyl-Gruppen 0,87) |
| 0,60 g | Hydroxypropylmethylcellulose |
| 0,50 g | Laurylpyridiniumchlorid |
| 96,80 g | Wasser, vollentsalzt |
| 100,00 g | (auf pH 5,0 mit 10-%-iger Ameisensäure eingestellt) |

Jeweils 35 g der Haarkurmittel nach Beispiel 9 beziehungsweise 10 wurden im gewaschenen Haar verteilt und nach einer Einwirkungszeit von 3 bis 5 Minuten mit Wasser wieder ausgespült; als Ergebnis wurde ein ausgezeichneter Griff, Glanz sowie Kämmbarkeit des Haares erhalten.

**Beispiel 11**

**Hautcreme**

| | |
|---|---|
| 0,30 g | quaternäres Chitosanderivat nach Beispiel 3 (η = 46 ml/g, Substitutionsgrad: kationische Gruppen 0,48; Hydroxyethyl-Gruppen 1,56) |
| 3,00 g | Stearylalkohol |
| 1,00 g | Wollfettalkohol (Adeps Lanae) |
| 1,00 g | Vaseline |
| 0,76 g | Milchsäure, 10-%-ig |
| 1,00 g | Natriumcetylstearylsulfat |
| 92,94 g | Wasser, vollentsalzt |
| 100,00 g | |

**Beispiel 12**

**Haartönungsmittel**

| | |
|---|---|
| 0,50 g | quaternäres Chitosanderivat nach Beispiel 2 (η = 513 ml/g, Substitutionsgrad: kationische Gruppen 0,48; Hydroxyethyl-Gruppen 3,1) |
| 12,00 g | Cetylstearylalkohol |
| 0,10 g | Parahydroxybenzoesäureethylester |
| 6,00 g | Laurylalkohol-diglycolethersulfat-Natriumsalz (28-%-ige wäßrige Lösung) |
| 0,50 g | Parfümöl |
| 79,31 g | Wasser |
| 0,05 g | 1-Hydroxy-2-amino-4-nitrobenzol (C.I. 76 530) |
| 0,85 g | 1,4-Diamino-2-nitro-benzol (C.I. 76 070) |
| 0,24 g | Natriumhydroxid |
| 100,00 g | |

Ungefähr 30 bis 40 g wurden in dem gewaschenen Haar verteilt und nach einer Einwirkungszeit von 20 Minuten ausgespült. Das Haar war rötlich gefärbt und wies eine gute Kämmbarkeit und einen angenehmen Griff auf.

**Beispiel 13**

**Oxidationshaarfärbemittel**

| | |
|---|---|
| 0,50 g | quaternäres Chitosanderivat nach Beispiel 2 (η = 513 ml/g, Substitutionsgrad: kationische Gruppen 0,48; Hydroxyethyl-Gruppen 3,1) |
| 0,08 g | 3,5-Diamino-2,6-dimethoxy-pyridin-dihydrochlorid |
| 0,30 g | 1,4-Diaminobenzol |
| 0,25 g | Resorcin |
| 0,30 g | Natriumsulfit |
| 3,50 g | Laurylalkohol-diglycolethersulfat-Natriumsalz (28-%-ige wäßrige Lösung) |
| 15,00 g | Cetylalkohol |
| 3,00 g | Ammoniak |
| 77,07 g | Wasser |
| 100,00 g | |

50 g dieses Haarfärbemittels wurden mit 50 ml 6-%-iger Wasserstoffperoxidlösung gemischt und auf weißes Haar aufgetragen. Nach 30 Minuten wurde das Haar mit Wasser ausgespült und getrocknet. Das Haar hat eine natürlich wirkende matt-blonde Färbung sowie einen natürlichen angenehmen Griff erhalten.

**Beispiel 14**

**Dauerwellmittel**

| | |
|---|---|
| 0,50 g | quaternäres Chitosanderivat nach Beispiel 3 (η = 46 ml/g, Substltutionsgrad: kationische Gruppen 0,48; Hydroxyethyl-Gruppen 1,56) |
| 10,00 g | Thioglykolsäure |
| 8,00 g | Ammoniak, 25-%-ig |
| 6,10 g | Ammoniumhydrogencarbonat |
| 75,40 g | Wasser |
| 100,00 g | |

Zur Anwendung trug man dieses Dauerwellmittel auf das gewickelte handtuchtrockene Haar gleichmäßig auf, und ließ es etwa 20 Minuten einwirken; danach wurde das Haar mit Wasser ausgespült und in bekannter Weise oxidativ behandelt. Es wurde ein gutes Wellergebnis erhalten, die Haare fühlten sich natürlich und weich an.

**Beispiel 15**

**Haarfestiger Alkoholfrei**

| | |
|---|---|
| 0,70 g | quaternäres Chitosanderivat nach Beispiel 3 ($\eta$ = 46 ml/g, Substitutionsgrad: kationische Gruppen 0,48; Hydroxyethyl-Gruppen 1,56) |
| 1,50 g | Ameisensäure 10-%-ig |
| 0,80 g | Parfüm |
| 0,10 g | Chloracetamid (Konservierungsmittel) |
| 96,90 g | Wasser, vollentsalzt |
| 100,00 g | |

20 ml dieser Lösung wurden auf die gewaschenen handtuchtrockenen Haare gegeben, sodann das Haar eingelegt und getrocknet. Bei guter Festigungswirkung zeigte das Haar einen angenehmen weichen Griff.

**Patentansprüche**

1. Kosmetisches Mittel zur Behandlung von Haaren oder der Haut, dadurch gekennzeichnet, daß es in einer geeigneten Kosmetikgrundlage eine quaternäre makromolekulare, vom Chitosan abgeleitete polymere Verbindung der allgemeinen Formel I

$$HO[C_6H_{11-m}NO_4(R^1)_m(R^2)_n(R^3)_q]_pH, \qquad (I)$$

wobei
m jeden beliebigen Zahlenwert von 0 bis 0,5 bedeutet,
n jeden beliebigen Zahlenwert von 0,01 bis 6 bedeutet,
q ein beliebiger Zahlenwert von 0,005 bis 3,0 ist,
p eine ganze Zahl von 10 bis 50 000 bedeutet,
$R^1$ ein Acetyl-Rest ist,
$R^2$ einen zweiwertigen Rest

$-CH_2-CH_2-O-$

darstellt und
$R^3$ ein zweiwertiger Rest

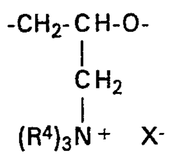

oder

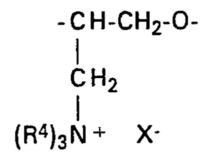

mit
$R^4 = C_1$- bis $C_4$-Alkyl und
X = Cl, Br, J oder $CH_3SO_4$ ist,
enthält.

2. Mittel nach Anspruch 1, dadurch gekennzeichnet, daß n in der Formel I einen Zahlenwert von 0,87 bis 3,1 bedeutet.

3. Mittel nach Anspruch 1 und 2, dadurch gekennzeichnet, daß q in der Formel I einen Zahlenwert von 0,42 bis 0,48 bedeutet.

4. Mittel nach Anspruch 1 bis 3, dadurch gekennzeichnet, daß es die polymere Verbindung der Formel I in einer Menge von 0,05 bis 10,0 Gew.-% enthält.

5. Mittel nach Anspruch 1 bis 4, dadurch gekennzeichnet, daß es als Kosmetikgrundlage eine wäßrige, alkoholische oder wäßrig-alkoholische Lösung, eine Creme, ein Gel oder eine Emulsion enthält.

6. Mittel nach Anspruch 1 bis 5, dadurch gekennzeichnet, daß es als Kosmetikgrundlage eine wäßrige oder wäßrig-alkoholische Lösung eines niedermolekularen Alkohols, wie Ethanol oder Isopropanol, umfaßt und einen pH-Wert zwischen 6 und 8 aufweist.

7. Mittel nach Anspruch 1 bis 5, dadurch gekennzeichnet, daß es zusätzlich ein kationisches, nicht-ionisches, amphoteres oder anionisches Tensid enthält und in Form eines Haarwaschmittels vorliegt.

8. Mittel nach Anspruch 7, dadurch gekennzeichnet, daß das anionische Tensid ein Alkylethersulfat ist.

9. Mittel nach Anspruch 7 und 8, dadurch gekennzeichnet, daß es das Tensid in einer Konzentration zwischen 3 und 25 Gew.-% enthält und einen pH-Wert zwischen 6 und 7 aufweist.

10. Mittel nach Anspruch 1 bis 6, dadurch gekennzeichnet, daß es als Kosmetikgrundlage eine alkoholische oder wäßrig-alkoholische Lösung enthält, die mit einem unter Druck verflüssigten Treibgas vermischt ist, in einem Druckbehälter abgefüllt ist und in Form eines Aerosolhaarsprays vorliegt.

11. Mittel nach Anspruch 1 bis 6, dadurch gekennzeichnet, daß die Kosmetikgrundlage eine wäßrige, alkoholische oder wäßrig-alkoholische Lösung, eine Creme, ein Gel oder eine Emulsion ist, in der das Chitosanderivat der Formel I in einer Konzentration zwischen 0,1 und 10 Gew.-% enthalten ist, und es in Form eines Hautbehandlungsmittels vorliegt.

12. Mittel nach Anspruch 1 bis 10, dadurch gekennzeichnet, daß es zusätzlich ein bekanntes filmbildendes synthetisches oder natürliches kosmetisches Polymer enthält.

13. Mittel nach Anspruch 1 bis 5, dadurch gekennzeichnet, daß die Kosmetikgrundlage eine wäßrige, alkoholische oder wäßrig-alkoholische Lösung oder eine Emulsion ist, welche zusätzlich einen Farbstoff enthält und in Form eines Farbfestigers oder Tönungsfestigers vorliegt.

14. Quaternäre makromolekulare, vom Chitosan abgeleitete polymere Verbindungen der allgemeinen Formel I

$$HO[C_6H_{11-m}NO_4(R^1)_m(R^2)_n(R^3)_q]_pH, \qquad (I)$$

wobei
m jeden beliebigen Zahlenwert von 0 bis 0,5 bedeutet,
n jeden beliebigen Zahlenwert von 0,01 bis 6 bedeutet,
q ein beliebiger Zahlenwert von 0,005 bis 3,0 ist,
p eine ganze Zahl von 10 bis 50 000 bedeutet,
$R^1$ ein Acetyl-Rest ist,
$R^2$ einen zweiwertigen Rest

$-CH_2-CH_2-O-$

darstellt und
$R^3$ ein zweiwertiger Rest

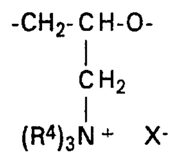
oder
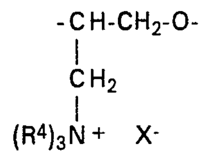

mit
$R^4 = C_1-C_4$-Alkyl und
$X = Cl, Br, J$ oder $CH_3SO_4$ ist.

15. Verbindung gemäß Anspruch 14, dadurch gekennzeichnet, daß in der Formel I n einen Zahlenwert von 0,87 bis 3,1 bedeutet, q einen Zahlenwert von 0,42 bis 0,48 darstellt und die Grenzviskositätszahl einen Wert von 46 bis 610 ml/g aufweist.

16. Verbindung gemäß Anspruch 14, dadurch gekennzeichnet, daß in der Formel I n = 1,56 bedeutet und q = 0,48 ist.

17. Verbindung gemäß Anspruch 14, dadurch gekennzeichnet, daß in der Formel I $R^4 = CH_3$ und $X = Cl$ bedeutet.

18. Verfahren zur Herstellung der Verbindungen nach Anspruch 14, dadurch gekennzeichnet, daß man ein Chitosan, bestehend aus zu 50 - 100 % entacetyliertem Chitin, mit einem Glycidyltrialkylammoniumhalogenid sowie zusätzlich mit Ethylenoxid in geeignetem Verhältnis gleichzeitig oder in beliebiger Reihenfolge zur Umsetzung bringt.

19. Verfahren nach Anspruch 18, dadurch gekennzeichnet, daß man das Chitosan in Gegenwart eines Lösungsmittels mit dem Glycidyltrialkylammoniumhalogenid und dem Ethylenoxid vermischt und diese Mischung bei einer Temperatur zwischen 10 und 100°C mehrere Stunden mischt oder rührt.

20. Verfahren nach Anspruch 18 und 19, dadurch gekennzeichnet, daß die Umsetzung in Gegenwart einer organischen oder anorganischen Base durchgeführt wird.

21. Verfahren nach Anspruch 18 und 19, dadurch gekennzeichnet, daß die Reaktion in Gegenwart einer organischen oder anorganischen Säure durchgeführt wird.

22. Verfahren nach Anspruch 18 bis 21, dadurch gekennzeichnet, daß man als Glycidyltrialkylammoniumhalogenid Glycidyltrimethylammoniumchlorid verwendet.

23. Verfahren nach Anspruch 18 bis 22, dadurch gekennzeichnet, daß man als Ausgangsmaterial durch Umfällen und Tiefgefrieren strukturell modifiziertes Chitosan verwendet.

24. Verfahren nach Anspruch 18 bis 23, dadurch gekennzeichnet, daß die Reaktion unter Druck im Autoklaven durchgeführt wird.

25. Verfahren nach Anspruch 18 bis 24, dadurch gekennzeichnet, daß man die Umsetzung in Gegenwart von Wasser durchführt.

## Claims

1. Cosmetic medium for treating hair or skin, characterised in that it contains, in a suitable cosmetic base, a quaternary macromolecular polymeric compound derived from chitosan, of the general formula I

$$HO[C_6H_{11-m}NO_4(R^1)_m(R^2)_n(R^3)_q]_pH \qquad (I),$$

wherein
m has any numerical value from 0 to 0.5,
n has any numerical value from 0.01 to 6,
q has any numerical value from 0.005 to 3,
p is an integer from 10 to 50000,
$R^1$ is an acetyl residue,
$R^2$ is a bivalent residue

$-CH_2-CH_2-O-$

and $R^3$ is a bivalent residue

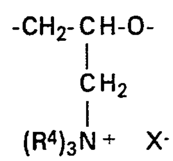

or

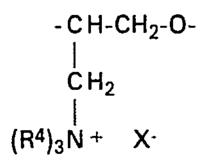

with
$R^4 = C_1$- to $C_4$-alkyl and
$X = Cl, Br, I$ or $CH_3SO_4$.

2. Medium according to Claim 1, characterised in that n in formula I has a numerical value from 0.87 to 3.1.

3. Medium according to Claim 1 and 2, characterised in that q in formula I has a numerical value from 0.42 to 0.48.

4. Medium according to Claim 1 to 3, characterised in that it contains the polymeric compound of formula I in a quantity of 0.05 to 10 weight %.

5. Medium according to Claim 1 to 4, characterised in that the cosmetic base is an aqueous, alcoholic or aqueous-alcoholic solution, a cream, a gel or an emulsion.

6. Medium according to Claim 1 to 5, characterised in that it contains as cosmetic base, an aqueous or aqueous-alcoholic solution of a low molecular weight alcohol, such as ethanol or isopropanol and has a pH value between 6 and 8.

7. Medium according to Claim 1 to 5, characterised in that it additionaly contains a cationic, non-ionogenic, amphoteric or anionic surfactant and is in the form of a hair shampoo.

8. Medium according to Claim 7, characterised in that the anionic surfactant is an alkylethersulphate.

9. Medium according to Claim 7 and 8, characterised in that it contains the surfactant in a concentration of between 3 and 25 weight % and has a pH-value of between 6 an 7.

10. Medium according to Claim 1 to 6, characterised in that the cosmetic base is an alcoholic or aqueous-alcoholic solution, which is mixed with a propellant gas liquefied under pressure, charged into a pressure container and is in the form of an aerosol hair spray.

11. Medium according to Claim 1 to 6, characterised in that the cosmetic base is an aqueous, alcoholic or aqueous-alcoholic solution, a creme, a gel or an emulsion, in which the chitosan derivative of formula I is contained in a concentration of between 0.1 and 10 weight %, and which is in the form of a skin treatment composition.

12. Medium according to Claim 1 to 10, characterised in that it also contains a known film-forming synthetic or natural cosmetic polymer.

13. Medium according to Claim 1 to 5, characterised in that the cosmetic base is an aqueous, alcoholic or aqueous-alcoholic solution or an emulsion, which also contains a dye and is in the form of a colour or tone strengthener.

14. Quaternary macromolecular polymeric compound derived from chitosan of the general formula I

$$HO[C_6H_{11-m}NO_4(R^1)_m(R^2)_n(R^3)_q]_pH \qquad (I),$$

wherein
m has any numerical value from 0 to 0.5,
n has any numerical value from 0.01 to 6,
q has any numerical value from 0.005 to 3.0,
p is an integer from 10 to 50000,
$R^1$ is an acetyl residue,
$R^2$ is a bivalent residue

$-CH_2-CH_2-O-$

and
$R^3$ is a bivalent residue

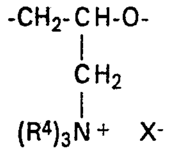
or
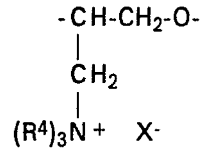

15. Compound according to Claim 14, characterised in that in formula I n has a numerical value between 0.87 and 3.1, q has a numerical value from 0.42 to 0.48 and the limiting viscosity number has a numerical value from 46 to 610 ml/g.

16. Compound according to Claim 14, characterised in that in formula I n is equal to 1.56 and q is equal to 0.48.

17. Compound according to Claim 14, characterised in that in formula I $R^4$ is $CH_3$ and X is Cl.

18. Process for preparing the compound according to Claim 14, characterised in that chitosan consisting of 50 to 100 % of deacetylated chitin is reacted with glycidyltrialkylammoniumhalide and ethylene oxide in a suitable ratio, simultaneous or in a given sequence.

19. Process according to Claim 18, characterised in that the chitosan is mixed with the glycidyltrialkylammoniumhalide and the ethylene oxide in the presence of a solvent and the resulting mixture is mixed or stirred for several hours at a temperature of between 10 and 100°C.

20. Process according to Claim 18 and 19, characterised in that the reaction is carried out in the presence of an organic or inorganic base.

21. Process according to Claim 18 and 19, characterised in that the reaction is carried out in the presence of an organic or inorganic acid.

22. Process according to Claim 18 to 21, characterised in that the glycidyltrialkylammoniumhalide is a glycidyltrimethylammoniumchloride.

23. Process according to Claim 18 to 22, characterised in that as basic material a chitosan is used which is structurally modified by reprecipitation and deep-freezing.

24. Process according to Claim 18 to 23, characterised in that the reaction is carried out in an autoclave under pressure.

25. Process according to Claim 18 to 24, characterised in that the reaction is carried out in the presence of water.

## Revendications

1. Produit cosmétique pour le traitement des cheveux ou de la peau, caractérisé en ce qu'il renferme, dans un substrat cosmétique approprié, un composé polymère quaternaire macromoléculaire dérivant du chitosane, répondant à la formule générale (I):

$$HO[C_6H_{11-m}NO_4(R^1)_m(R^2)_n(R^3)_q]_pH \qquad (I)$$

m représentant n'importe quelle valeur numérique de 0 à 0,5,
n représentant n'importe quelle valeur numérique de 0,01 à 6,
q représentant une valeur numérique quelconque de 0,005 à 3,0,
p représentant un nombre entier de 10 à 50.000,
$R^1$ étant un reste acétyle,
$R^2$ représentant un reste bivalent

$-CH_2-CH_2-O-$

et
$R^3$ représentant un reste bivalent

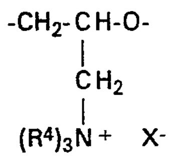

ou

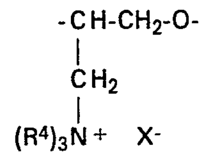

avec
$R^4$ = alkyle en $C_1$ à $C_4$ et
X = Cl, Br, I ou $CH_3SO_4$.

2. Produit selon la revendication 1, caractérisé en ce que, dans la formule (I), n représente une valeur numérique de 0,87 à 3,1.

3. Produit selon les revendications 1 et 2, caractérisé en ce que, dans la formule (I) q représente une valeur numérique de 0,42 à 0,48.

4. Produit selon les revendications 1 à 3, caractérisé en ce qu'il renferme le composé polymère répondant à la formule (I) dans une proporiton de 0,05 à 10,0 % en poids.

5. Produit selon les revendications 1 à 4, caractérisé en ce qu'il renferme, comme substrat cosmétique, une solution aqueuse, alcoolique ou hydro-alcoolique, une crème, un gel ou une émulsion.

6. Produit selon les revendications 1 à 5, caractérisé en ce qu'il renferme, comme substrat cosmétique, une solution aqueuse ou hydro-alcoolique d'un alcool inférieur, comme l'éthanol ou l'isopropanol, et présente une valeur de pH comprise entre 6 et 8.

7. Produit selon les revendications 1 à 5, caractérisé en ce qu'il renferme en plus un agent tensio-actif cationique, non-ionique, amphotère ou anionique et se présente sous la forme d'un produit de lavage des cheveux.

8. Produit selon la revendication 7, caractérisé en ce que l'agent tensio-actif anionique est un éther-sulfate d'alkyle.

9. Produit selon les revendications 7 et 8, caractérisé en ce qu'il renferme l'agent tensio-actif à une concentration comprise entre 3 et 25 % en poids et présente une valeur de pH comprise entre 6 et 7.

10. Produit selon les revendications 1 à 6, caractérisé en ce qu'il renferme, comme substrat cosmétique, une solution alcoolique ou hydro-alcoolique mélangée avec un gaz propulseur liquéfié sous pression, transvasée dans un réservoir sous pression et se présentant sous la forme d'un spray capillaire aérosol.

11. Produit selon les revendications 1 à 6, caractérisé en ce que le substrat cosmétique est une solution aqueuse, alcoolique ou hydro-alcoolique, une crème, un gel ou une émulsion, où le dérivé du chitosane répondant à la formule (I) est contenu à une concentration comprise entre 0,1 et 10 % en poids, et en ce qu'il se présente sous la forme d'un produit de traitement de la peau.

12. Produit selon la revendication 1 à 10, caractérisé en ce qu'il renferme, en plus, un polymère cosmétique synthétique ou naturel filmogène connu.

13. Produit selon les revendications 1 à 5, caractérisé en ce que le substrat cosmétique est une solution aqueuse, alcoolique ou hydro-alcoolique ou une émulsion qui renferme en plus un colorant et se présente sous la forme d'un fixateur de couleur ou d'un fixateur de nuance.

14. Composés polymères quaternaires macromoléculaires dérivant du chitosane, répondant à la formule générale (I)

$$HO[C_6H_{11-m}NO_4(R^1)_m(R^2)_n(R^3)_q]_pH \qquad (I)$$

m représentant n'importe quelle valeur numérique de 0 à 0,5,
n représentant n'importe quelle valeur numérique de 0,01 à 6,
q étant une valeur numérique quelconque de 0,005 à 3,0,
p représentant un nombre entier de 10 à 50.000,
$R^1$ étant un reste acétyle,
$R^2$ représentant un reste bivalent

$-CH_2-CH_2-O-$

et
$R^3$ représentant un reste bivalent

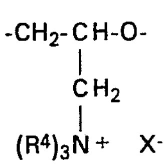

ou

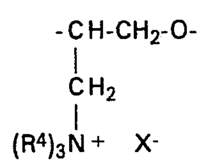

avec

$R^4$ = alkyle en $C_1$ à $C_4$ et
X = Cl, Br, I ou $CH_3SO_4$.

15. Composé selon la revendication 14, caractérisé en ce que, dans la formule (I), n représente une valeur numérique de 0,87 à 3,1, q représente une valeur numérique de 0,42 à 0,48 et en ce que l'indice de viscosité-limite présente une valeur de 46 à 610 ml/g.

16. Composé selon la revendication 14, caractérisé en ce que, dans la formule (I), n = 1,56 et q = 0,48.

17. Composé selon la revendication 14, caractérisé en ce que, dans la formule (I), $R^4$ = $CH_3$ et X = Cl.

18. Procede de préparation des composés selon la revendicatien 14, caractérisé en ce qu'on fait réagir un chitosane comprenant de la chitine désacétylée à 50 à 100 % sur un halogénure de glycidyl-trialkylammonium, ainsi en plus que sur de l'oxyde d'éthylène dans une proportion appropriée, en même temps ou dans un ordre quelconque.

19. Procédé selon la revendication 18, caractérisé en ce qu'on mélange le chitosane, en présence d'un solvant, avec l'halogénure de glycidyltrialkylammonium et l'oxyde d'éthyléne, et l'on mélange ou l'on agite ce mélange plusieurs heures, à une température comprise entre 10 et 100°C.

20. Procédé selon les revendications 18 et 19, caractérisé en ce qu'on effectue la réaction en présence d'une base organique du inorganique.

21. Procédé selon les revendications 18 et 19, caractérisé en ce qu'on effectue la réaction en présence d'un acide organique ou inorganioue.

22. Procédé selon les revendications 18 à 21, caractérisé en ce qu'on utilise, comme halogénure de glycidyl-trialkylammonium, du chlorure de glycidyltriméthylammonium.

23. Procédé selon les revendications 18 à 22, caractérisé en ce qu'on utilise, comme matière de départ, du chitosane à structure modifiée par reprécipitation et congélation.

24. Procédé selon les revendications 18 à 23, caractérisé en ce qu'on effectue la réaction sous pression à l'autoclave.

25. Procédé selon les revendications 18 à 24, caractérisé en ce qu'on effectue la réaction en présence d'eau.